# EUROPEAN PATENT SPECIFICATION

(11) **EP 2 233 501 B1**
(45) Date of publication and mention of the grant of the patent: **11.07.2012**
(21) Application number: 08865354.8
(22) Date of filing: 03.12.2008
(51) Int. Cl.: C07K 16/28, C12P 21/08, G01N 33/543, A61K 39/44, A61P 7/06, A61P 35/00, C12N 15/13, G01N 33/574, G01N 33/569

(54) **HUMANIZED ANTI-CD34 MONOCLONAL ANTIBODY, THE PREPARATION AND USES THEREOF**
HUMANISIERTER MONOKLONALER ANTI-CD34-ANTIKÖRPER UND DESSEN HERSTELLUNG UND ANWENDUNGEN
ANTICORPS MONOCLONAL ANTI-CD34 HUMANISÉ, SA PRÉPARATION ET SES UTILISATIONS

(30) Priority: 13.12.2007 CN 200710094456; 08.01.2008 CN 200810043016
(43) Date of publication of application: 29.09.2010
(73) Proprietor: Shanghai Guojian Bio-Tech Institute, Shanghai 201203 (CN)
(72) Inventor: GUO, Yajun, Shanghai 201203 (CN); QIAN, Weizhu, Shanghai 201203 (CN); HOU, Sheng, Shanghai 201203 (CN); LI, Bohua, Shanghai 201203 (CN); WANG, Hao, Shanghai 201203 (CN); MA, Jing, Shanghai 201203 (CN)
(74) Representative: Høiberg A/S
(86) International application number: PCT/CN2008/001963
(87) International publication number: WO 2009/079922

(56) References cited:
- EP-A1- 1 083 226
- CN-A- 101 245 108
- US-A1- 2007 207 210
- W. QIAN ET AL.: "Development of new versions of anti-human CD34 monoclonal antibodies with potentially reduced immunogenicity." BIOCHEMICAL AND BIOPHYSICAL RESEARCH COMMUNICATIONS, vol. 367, no. 2, 7 March 2008 (2008-03-07) , pages 497-502, XP027016399 U.S.A.
- S. HOU ET AL.: "Humanization of an anti-CD34 monoclonal antibody by complementarity-determining region grafting based on computer-assisted molecular modelling." JOURNAL OF BIOCHEMISTRY, vol. 144, no. 1, July 2008 (2008-07), pages 115-120, XP002605633 Japan
- HOU S ET AL.: 'Humanization of an anti-CD34 monoclonal antibody by complementarity-determining region grafting based on computer-assisted molecular modeling' J BIOCHEM vol. 144, no. 1, July 2008, pages 115 - 120, XP002605633
- DATABASE GENBANK 19 February 2008 'anti-human CD34 mAb 4C8 immunoglobulin heavy chain variable region ?Mus musculus?' Database accession no. ABY61978
- DATABASE GENBANK 19 February 2008 'anti-human CD34 mAb 4C8 immunoglobulin light chain variable region ?Mus musculus?' Database accession no. ABY61979
- LI BO HUA: 'Preparation and Characterization of an Anti-human CD3 Humanized Antibody' DATABASE OF CHINESE DOCTOR'S DEGREE THESIS, 31 January 2003,
- LI AI LING: 'Preparation and Functional Study of Monoclonal Antibodies against the Human CD20 and CD34,' DATABASE OF CHINESE DOCTOR'S DEGREE THESIS 31 January 2003,

## Description

### FIELD OF THE INVENTION

The present invention relates to biotechnology, and in particular to an antibody, the preparation method and uses thereof.

### BACKGROUND OF THE INVENTION

Tranplantation of hematopoietic stem/progenitor cells (HSCs) has currently become well-recongnized as the only effective treatment method for certain malignant blood diseases and solid tumors. However, because of numerous T lymphocyte contained in the grafts, acute, severe graft versus-host disease (GVHD) occurs after grafting, which is one of the major cause that affect whether the heterologous genetic hematopoietic stem cells grafting can survival long or not. The primary problem of tranplantation of hematopoietic stem/progenitor cells is how to obtain great amount of purified hematopoietic stem cells to remove the interference of the impurity cells in grafts. Hence, to purge or enrich specific cells in the grafts is a problem concerned in present grafting field. Since HSCs have no definite morphological features, but mainly perform as lymphocyte-like mononuclear blast cells, they can only be indentified by some proteins on the cell surface. CD34, a transmembrane glycoprotein of 110 kDa, is the broadest and earliest antigen so far recognized as expressed on human HSC, and is a good marker to evaluate the quality and quantity of HSC. With the development of CD34 antibody-based positive selection technology, CD34+ hematopoietic stem cells exhibit to reconstitute of the hematopoietic function on experiment animals and human beings. *[*Andrews RG, Bryant EM, Bartelmez SH, Muirhead DT, Knitter GH, Bensinger W, Strong DM, Bernstien ID(1992)CD34+ marrow cells, devoid of T and B Lymphocytes, reconstitute stable lymphopoiesis and myelopoiesis in lethally irradiated baboons. Blood 80:1693*] [*Shpall EJ, Jones RB, Franklin W, Bearman S, Stemmer S, Hami L, Petsche D, Taffs S, Myers S, Purdy M, Heimfeld S, Halligan J, Berenson RJ(1994)Transplantation of autologous CD34+ hematopoietic progenitor cells into breast cancer patient following high-dose chemotherapy. J Clin Oncol 12:28*],* hence, they have become an optimal target cell for hematopoietic stem cells grafting due to their unique biological characteristics and function.

Currently, the mostly used methods for cell separation and purification included adhesion, differential centrifugation, discontinuous density gradient method and the like. Although these technologies have solved many practical problems, they hinder the progress of cell biology and the development and application of cell biotechnology product due to long operation period, low cell recovery rate, and high cost for separation and purification. Immunomagnetic cell sorting technology is a recently developed new cell separation technology, which has advantages of simple and easy to operate, high separation purity, and retaining the cell activity, etc. and can be used in separating and examining various marrow and blood cells, tumor cells, bacteria and other microorganisms, etc. Thus, it is favored by researchers of biomedical field. Recently, immunomegatic beads are developed gradually towards submicron size, which allows the magnetic beads to separate cells easily and rapidly at low gradient magnetic field, and to perform subsequent analysis and application directly without dissociation of the magnetic beads from cell surface, and the beads have no effect on the cell activities after separation. The principle of separation is: using magnetic nano particles as vector to conjugate the antibody on their surfaces, due to the specific affinity of antibody, the antibody on the magnetic nano particles binds the specific surface antigen on the cells to form cell-antibody-magnetic nano particles complex, under the oriented control of applied magnetic field, target cells can be isolated directly from original cell mixture in one step by the operation of affinity adsorption, washing and desorption, etc. Nano magnetic cells affinity separation has dual advantages, easily and conveniently magnetic separation and high selectivity of affinity separation.

EP1083226 discloses chimeric mouse-human mAb anti-CD34, based on murine mAb My10, which can be attached to magnetic Dyna-beads for isolation of CD34-positive cells.

With the coming out of immunomagnetic beads, use of immunomagnetic beads, which is prepared by the conjugation of CD34 monoclonal antibody to magnetic nano microspheres for sorting marrow hematopoietic stem cells has been applied to clinical hematopoietic stem cells transplantation. Owing to the fact that magnetic separation has advantages of easy and convenient operation, rapid and complete separation, and high cell purity, etc when sorting cells, HSC transplantation has become the first choice for certain malignant hematological diseases, severe immunodeficiency and low hematopoietic function resulted from radiotherapy and chemotherapy for tumors. During the course of hematopoietic stem cell transplantation, these antibodies that bind to the magnetic beads will inevitably enter into human body along hematopoietic stem cells. Murine-derived monoclonal antibody produced by hybridoma technology may cause human anti-mouse antibody response (HAMA) after entering human body *[*Winter G, Harris WJ. Humanized antibodies. Immunol Today. 1993 Jun; 14(6): 243-6*].* Hence, it is an urgent problem for those skilled in the art to construct a low immunogenicity antibody which can reduce the murine component, and effectively reduce the incidence rate of HAMA by genetic engineering technology.

Humanized antibody is a new genetic engineering antibody developed to overcome the deficiency of murine derived monoclonal antibody. The first generation of humanized antibody is a chimeric antibody composed of a variable region of murine monoclonal antibody and a constant region of human antibody. Since the affinity between antibody and antigen is determined by its variable region, chimeric antibody keeps good affinity, meanwhile, the immunogenicity is reduced to a certain extent. The variable regions of antibody are composed of hypervariable regions (CDRs) and frame regions (FRs), wherein CDRs are highly variable regions which mediate directly the binding of antibody to antigen, FR regions are relative conserved, and are used as support to maintain the spatial position of CDR regions which are the main regions to produce immunogenicity in the variable regions. Since chimeric antibody retains the variable region of murine derived antibody, it may still cause strong HAMA reaction in clinical application. Therefore, in order to reduce the immunogenicity of chimeric antibody to the greatest extent, people consider grafting directly murine CDR region to FR region of human derived antibody variable region, yielding CDR graft antibody, namely humanized antibody. However, the transfer of murine CDRs alone usually results in a significant loss of antigen binding, because certain framework residues are critical for preserving the CDR conformations or are directly involved in antigen binding. In most cases, the successful design of humanized antibodies requires that these key murine residues be reintroduced into the human framework to restore affinity. Hence, it is critical for antibody humanization of how to determine the important residues that affect the antibody activity in FR.

### SUMMARY OF THE INVENTION

An object of the present invention is to provide a humanized anti-human CD34 antibody, an antibody keeping good affinity obtained by grafting a murine CDR region into a humanized antibody framework region, and at the same time reshaping the amino acids of the humanized antibody framework region.

Accordingly, the present invention provides a humanized anti-human CD34 antibody, said antibody comprises at least one antigen binding site, said antigen binding site at least comprises:
A first variable region having an amino acid sequence as shown in amino acids 1-122 of SEQ ID NO: 6; and
A second variable region having an amino acid sequence as shown in amino acids 1-113 of SEQ ID NO: 8.

The above first variable region and second variable region correspond to heavy chain variable region and light chain variable region of immunoglobulin, respectively, wherein, a hypervariable regions (CDR) of the first variable region sequentially comprises CDR1, CDR2 and CDR3, of which the amino acid sequences are CDR1: GYTFTNYGMN (SEQ ID NO:14); CDR2: WINTNTGEPKYAEEFKG (SEQ ID NO:15) and CDR3: GYGNYARGAWLAY (SEQ ID NO:16), respectively. Besides the above CDR regions, the first variable region further comprises a framework region (FR) derived from human immunoglobulin heavy chain variable region. In order to improve the affinity of the antibody, the amino acids of the framework region have been partially reshaped in the present invention, thereby obtaining the first variable region having the amino acid sequence of SEQ ID NO: 6, with SEQ ID NO: 5 being the corresponding nucleotide sequence encoding the same. Similarly, a hypervariable regions (CDR) of the second variable region sequentially comprises CDR1', CDR2' and CDR3', of which the amino acid sequences are CDR1': RSSQTIVHSNGNTYLE (SEQ ID NO:17); CDR2': QVSNRFS (SEQ ID NO:18) and CDR3': FQGSHVPRT (SEQ ID NO:19), respectively. Besides the above CDR regions, the second variable region further comprises a framework region (FR) derived from the human immunoglobulin light chain variable region. In order to improve the affinity of the antibody, the amino acids of the framework region have been partially reshaped in the present invention, thereby obtaining the second variable region having the amino acid sequence of SEQ ID NO: 8, with SEQ ID NO: 7 being the corresponding nucleotide sequence encoding the same.

Further, in the humanized anti-human CD34 antibody of the present invention, said antibody comprises:
1) an immunoglobulin heavy chain or fragment thereof, comprising said first variable region and human immunoglobulin heavy chain constant region or fragment thereof; and
2) an immunoglobulin light chain or fragment thereof, comprising said second variable region and human immunoglobulin light chain constant region or fragment thereof.

The human immunoglobulin constant region can be selected from various types of immunoglobulin, preferably, the human immunoglobulin heavy chain constant region or fragment thereof is type γ (IgG1) having the amino acid sequence as shown in SEQ ID NO: 2; the human light chain constant region or fragment thereof is type κ or type λ, a preferable amino acid sequence of the light chain constant region is shown in SEQ ID NO: 4.

Another object of the present invention is to provide a nucleotide molecule encoding the above-mentioned humanized human CD34 antibody, a host transformed by the nucleotide molecule is also included in the present invention.

The present invention further provides a preparation method for the above-mentioned humanized anti-CD34 antibody, comprising designing an amino acid sequence of a humanized antibody h4C8 based on computer-assisted molecular modeling, performing whole gene synthesis of the heavy chain and light chain variable region genes and splicing the same with human antibody heavy and light chain constant regions, respectively though genetic recombination, cloning into eukaryotic expression vectors to construct expression vectors for humanized antibody heavy and light chain, then co-transfecting the expression vectors of light and heavy chain into CHO cells by liposome method, and then screening, culturing and purifying to obtain the product.

A serial of experiments have been carried out in the present invention using the resultant antibody, the result of antigen-binding activities assay *in vitro* indicates that h4C8 can bind specifically to human myeloid leukemia cell KG-1a which highly expressed antigen CD34. The result of competitive inhibition experiment indicates that h4C8 retains the affinity and specificity of original murine-derived antibody. The antibody can be conjugated with nano magnetic materials to prepare immunomagnetic beads to screen marrow hemopoietic stem cells. It may effectively reduce the incidence rate of HAMA and improve the security of clinical transplantation of hematopoietic stem cells and can be used in the treatment of some malignant hematologic diseases and solid tumors.

A method of carbodiimide/N-Hydroxysuccinimide (EDC/NHS) is used to activate the carboxyl group on the surface of the magnetic beads, the activated carboxyl group then reacts with the amino group on the antibody, conjugates CD34 antibody, and prepares CD34+ immunomagnetic beads that can effectively isolate cells. A sorting model is prepared using CD34+ KG-1a cells and CD34- Raji cells, and CD34 monoclonal immunomangetic beads are used to sort CD34+ cells. Also, CD34+ immunomagnetic nano particles are used to sort umbilical cord blood stem cell, and the purity and yield of the cells obtained by sorting are analyzed.

Anti-CD34 monoclonal antibody binding to magnetic nano particles is used as antibody vector to binds CD34+ hematopoietic stem/progenitor cells and thus forms immunocomplex. Under the effect of applied magnetic field, the complex moves dynamically and isolates the hematopoietic stem/progenitor cells that specifically bind the antibody on the magnetic nano particles from other cells (tumor cells that do not express CD34 antigen). The purified hematopoietic stem cells are transfused back to patients to reconstruct the hematopoietic and immune systems and effectively reduce the recurrence of tumors.

### BRIEF DESCRIPTION OF DRAWINGS

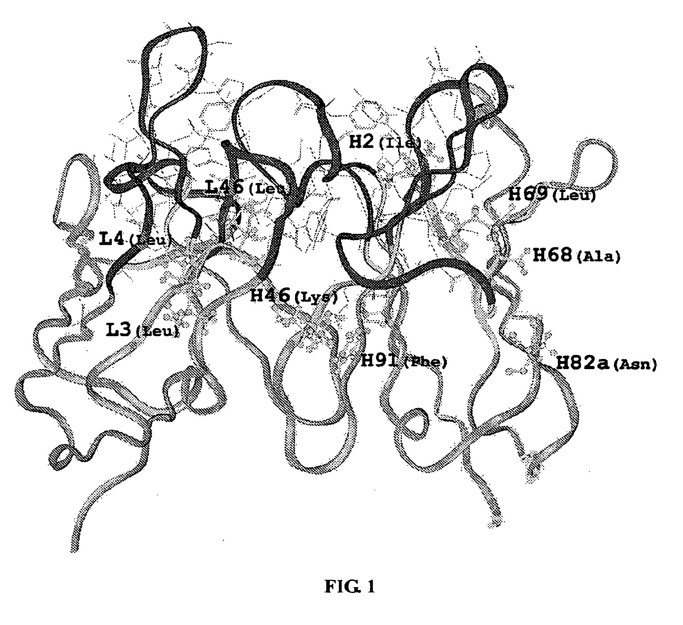

Figure 1 is a schematic view of molecular simulation structure of 4C8 monoclonal antibody: the FR region residues are represented by light grey ribbon, CDR region residues are represented by dark grey ribbon, nine FR region residues within 5 angstrom (Å) around CDR regions are represented by light grey ball-and-stick;

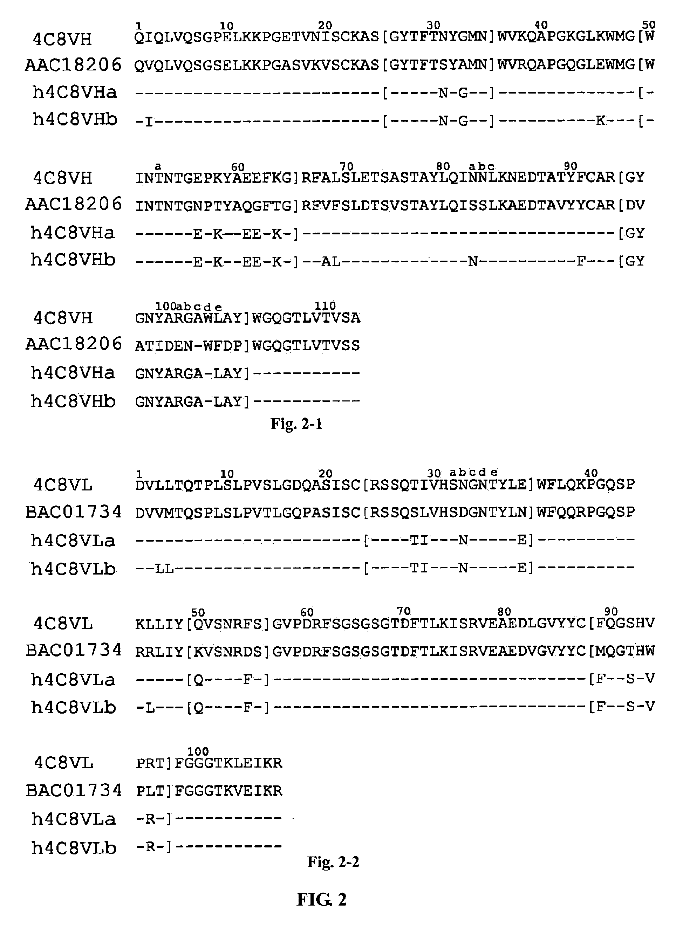

Figure 2 is an alignment view of amino acid sequences of heavy chain (Fig. 2-1) and light chain (Fig. 2-2) of a humanized antibody h4C8 with relevant sequences, wherein 4C8VH and 4C8VL represent the heavy chain and light chain variable regions of murine-derived monoclonal antibody 4C8, respectively; the heavy chain variable region of human antibody AAC18206 and the light chain variable region of human antibody BAC01734 are selected as the framework regions of the heavy chain and light chain of humanized antibody hu4C8, respectively; hu4C8VHa and hu4C8VHb represent heavy chain variable regions of different humanized antibody, h4C8VLa and h4C8VLb represent light chain variable regions of different humanized antibody, respectively; a dash represents an amino acid which is identical to the corresponding residue of human antibody AAC18206 or BAC01734, CDR regions are represented in the bracket; the amino acids are numbered according to the Kabat numbering manner *[*E.A. Kabat, T.T. Wu, H.M. Perry, K.S. Gottesman, C. Foeller, Sequences of Proteins of Immunological Interest, Fifth ed, United States Department of Health and Human Services, Bethesda, MD, 1991*.];*

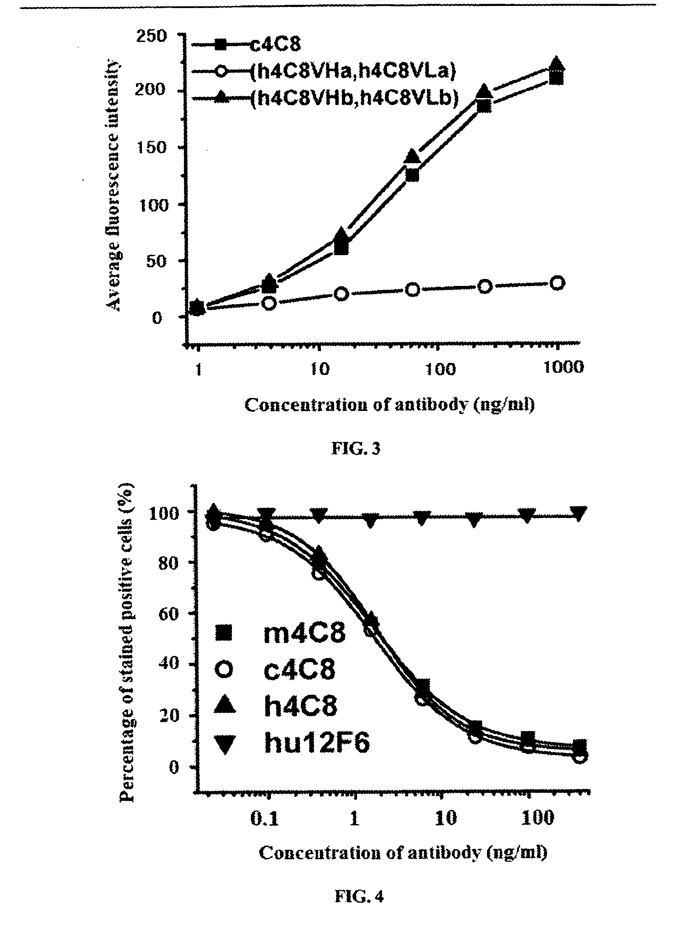

Figure 3 is the result of antigen-binding activities assay of humanized 4C8 antibody;
Figure 4 is the result of competitive inhibition experiment;

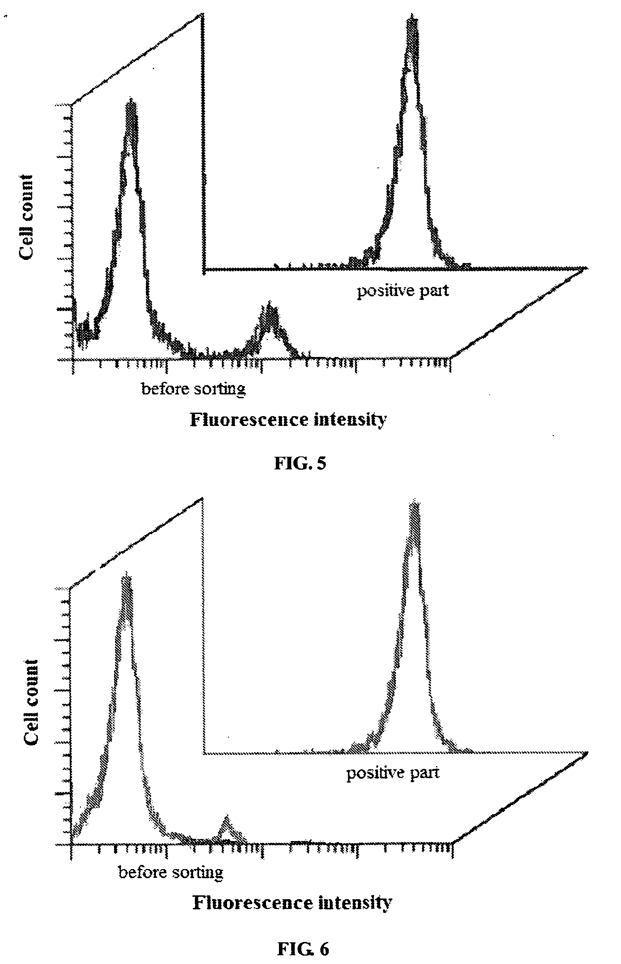

Figure 5 is the result of CD34+ cell lines sorting experiment using CD34 immunomagnetic beads: front side is the mixed cells before sorting, peak at the left side is CD34- Raji cells, peak at the right side is CD34+ KG-1a cells; back side is positive cell populations after sorting, wherein CD34- Raji cells have been removed to effectively enrich the CD34+ cells;
Figure 6 is the result of human umbilical cord blood stem cell sorting experiment using CD34 immunomagnetic beads: front side is the umbilical cord blood mononuclear cell before sorting, peak at the left side is CD34- cell populations, peak at the right side is CD34+ cell populations; back side is positive cell populations after sorting, wherein CD34- cells have been removed to effectively enrich the CD34+ stem cells;

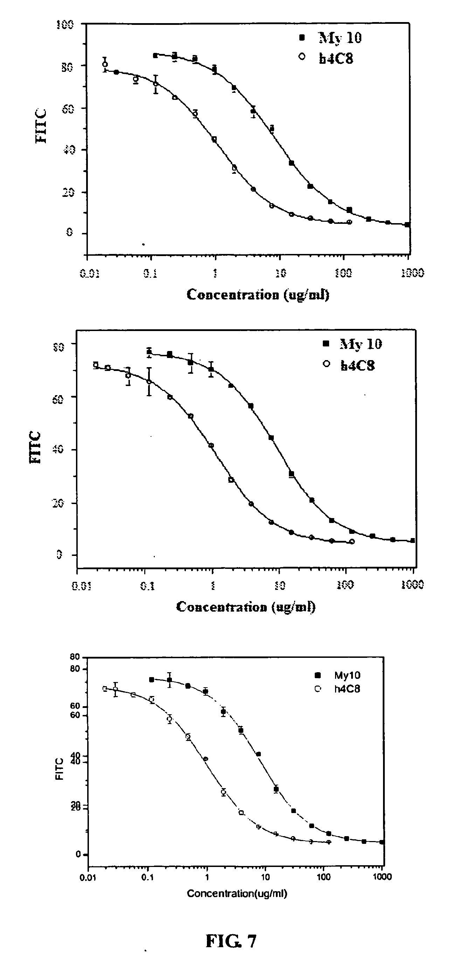

Figure 7 is the result of competitive inhibition experiment of humanized anti-CD34 antibody with murine-derived antibody My 10;

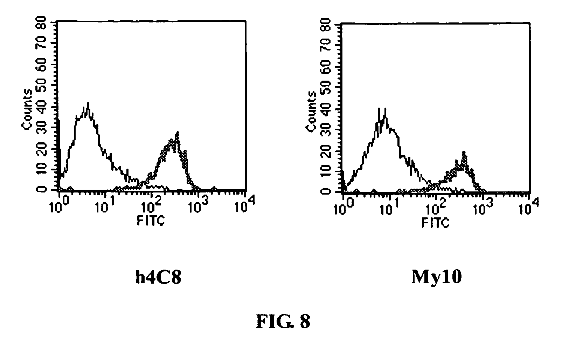

Figure 8 is the comparative experimental result of umbilical cord blood stem cells sorting after humanized anti-CD34 antibody and murine-derived antibody My10 have conjugated with beads (peak at left side is test result before sorting, and peak at right side is test result after sorting).

### DETAIL DESCRIPTION OF THE INVENTION

The following examples are just further description of the present invention and should not be understood as the limitation to the present invention.

KG-1a (human leukaemia cell, ATCC, CCL-246.1)

Raji (human B lymphoma cell, ATCC, CCL-86)

In the present invention, 4C8 refers to that obtained by the method disclosed in the invention published as Chinese invention patent application No. 200710094456.9 on August 20, 2008, entitled anti-human CD34 antibody, the preparation method and uses thereof, based on the application filed on December 13, 2007 by the present applicant, the method of which will be described in details below.

### Cloning of Genes Encoding Human Antibody Light and Heavy Chain Constant Region

Healthy human lymphoma cells were isolated from lymphoma cells separation solution (Dingguo Biotechnology Development Company, CHINA), total RNA was extracted using Trizol reagent (Invitrogen), the genes encoding antibody heavy chain and light chain constant region were amplified by RT-PCR reaction, with the primers designed according to the sequences reported in the reference (Cell, 1980, 22: 197-207) and reference (Nucleic Acids Research, 1982, 10: 4071-4079), respectively. The PCR products were purified by agarose gel electrophoresis and recovered and cloned into pGEM-T vector, correct clones were obtained by sequencing verification. SEQ ID NO:1 and SEQ ID NO:2 show the nucleotide sequence and amino acid sequence of the heavy chain constant regions (CH), respectively. SEQ ID NO:3 and SEQ ID NO:4 show the nucleotide sequence and amino acid sequence of the light chain constant regions (CL), respectively. In this example, the correct clones were marked as pGEM-T/CH and pGEM-T/CL.

### Example 1. Preparation of anti-human CD34 monoclonal antibody 4C8---preparation of monoclonal antibody by cell fused hybridoma

BALB/c mice (obtained from SHANGHAI LABORATORY ANIMAL CENTER) were immunized with KG-1a cells which highly express CD34 (KG-1a cell ATCC CCL-246.1), allowing the B lymphocytes cells in the spleen to produce anti-human CD34 antibody, the splenocytes from immunized mice were fused with NS-1 (BALB/c mice myeloma cells), selectively cultured by HAT, after culturing, the anti-human CD34 positive clones were selected and after cloning, the sub-clones were selected to ensure the antibody is produced by single cloning cell, then culture supernatants from each cloning cell were collected , and the anti-human CD34 monoclonal antibody 4C8 was obtained by Protein G column purification.

### Example 2. Construction of chimeric antibody c4C8

### Cloning of Gene Encoding Anti-human CD34 Monoclonal Antibody 4C8 Variable Region

Total RNA was extracted from 2× 10⁶ hybridoma cell 4C8 which secrets anti-human CD34 with "Trizol Reagent" reagent kit (Gibco BRL, USA) according to its instructions. Three gene specific primers GSP1, GSP2, GSP3 were designed respectively by selecting the proper positions of antibody (IgG1,κ) heavy chain and light chain constant regions, wherein, GSP1 is apart furthest from the variable region gene, and used for reverse transcription reaction, GSP2 is used for first run PCR amplification, GSP3 is used for nest amplification. The primers were synthesized by SHANGHAI SANGON BIOLOGICAL TECHNOLOGY & SERVICES CO., LTD, the sequences of which were as follows: GSP1-H, 5'-GTA GAG GTC AGA CTG CAG GAC-3' (SEQ ID NO:20); GSP2-H, 5'-CTC AGG GAA ATA GCC CTT GAC-3' (SEQ ID NO:21); GSP3-H, 5'-AGA TCC AGG GGC CAG TGG ATA GAC-3' (SEQ ID NO:22). GSP1-L, 5'-TTG CTG TCC TGA TCA GTC CAA CT-3' (SEQ ID NO:23); GSP2-L, 5'-TGT CGT TCA CTG CCA TCA ATC TT-3' (SEQ ID NO:24); GSP3-L, 5'-TTG TTC AAG AAG CAC ACG ACT GA-3' (SEQ ID NO:25).

Total RNA was reverse transcribed into cDNA using GSP1 as primer according to the instruction of 5'RACE reagent kit (Gibco BRL, US), then poly(C) tail was added to the 3' end of first chain cDNA, after tailing, PCR amplification was carried out using GSP2 and AAP as primers, the amplification product was diluted 100 times and nest PCR amplification was carried out using AUAP and GSP3 as primer. Both PCR reactions were hot-start PCR, the reaction conditions were: 94°C for 5 minutes (min); 94°C for 45 seconds (sec), 60°C for 45 seconds, 72°C for 70 seconds, for 30 cycles; 72°C for 7 minutes. The products of nest PCR were separated by 1% agarose gel electrophoresis, then the fragment of interest was recovered and purified, and cloned into pGEM-T easy vector, the positive clones were selected and sequenced, and analysis was carried out to the sequencing results. Then the correct pGEM-T/V_{H} confirmed by sequencing was taken as template to design the primers H sense AAG CTT GCC GCC ACC ATG GAT TGG CTG TGG AAC TTG (SEQ ID NO:26) and H antisense GCT AGC TGC AGA GAC AGT GAC CAG (SEQ ID NO:27), VH chain variable region genes were amplified using Onestep RT-PCR reaction to make its 5' end contain restriction enzyme site HindIII, and 3' end contain restriction enzyme site Nhe I, the reaction conditions were: 50°C for 30 min; 95°C for 15 min; 94°C for 50 sec, 58°C for 50 sec, 72°C for 50 sec, for 30 cycles; 72°C for 10 min. The PCR products were purified by agarose gel electrophoresis and recovered and cloned into pGEM-T vector (Promega), the positive clones were selected and verified by sequencing, the result demonstrated that the sequence coincides completely with the sequence of 5'RACE. SEQ ID NO:9 and SEQ ID NO:10 show the nucleotide sequence and amino acid sequence of 4C8 heavy chain variable region, respectively. In this example, the correct clones were designated as pGEM-T/VH.

Taking the correct pGEM-T/V_{L} verified by sequencing as template to design the primers H sense AAG CTT GCC GCC ACC ATG AAG TTG CCT GTT AGG CTG (SEQ ID NO:28) and L antisense GAC AGA TGG TGC AGC CAC AGT CCG TTT GAT TTC CAG CTT G (SEQ ID NO:29), VL gene was amplified using Onestep RT-PCR reaction to make its 5' end contain restriction enzyme site HindIII, and 3' end contain the complementary sequence of 5' end of human antibody light chain constant region, the reaction conditions were: 94°C for 5 min; 94°C for 50 sec, 58°C for 50 sec, 72°C for 1 min, for 30 cycles; 72°C for 10min. The PCR products were purified by agarose gel electrophoresis and recovered and cloned into pGEM-T vector (Promega), the positive clones were selected and verified by sequencing, the result demonstrated that the sequence coincides completely with the sequence of 5'RACE. SEQ ID NO:11 and SEQ ID NO:12 show the nucleotide sequence and amino acid sequence of 4C8 light chain variable region, respectively. In this example, the correct clones were designated as pGEM-T/VL.

### Construction of Chimeric Antibody c4C8

The above plasmid pGEM-T/VH which was sequenced as correct after Onestep RT-PCR was digested by HindIII and NheI, 430bp digested fragment which was obtained after agarose gel electrophoresis purification and recovery was ligated to plasmid pGEM-T/ CH which was also digested by the same enzymes, the correct clones were selected and digested by HindIII and EcoR I, after agarose gel electrophoresis purification, the fragment of interest was recovered and ligated to pcDNA3.1 (+) (Invitrogen, US) which was digested by the same enzyme using T4 DNA ligase (Invitrogen, US), to construct eukaryotic expression vector pcDNA3.1 (+) (VHCH).

The above plasmid pGEM-T/VL which was sequenced as correct after Onestep RT-PCR was fused directly to the light chain constant region of correct clone pGEM-T/VL using Overlapping PCR, the reaction conditions were: 50°C for 30 min; 95°C for 15 min; 94°C for 50 sec, 58°C for 50 sec, 72°C for 50 sec, for 30 cycles; 72°C for 10 min, yielding a PCR product VLCL, the 5' end of which contains restriction enzyme site HindIII, and the 3' end contains restriction enzyme site EcoR I. After agarose gel electrophoresis purification, the PCR product was recovered and cloned into pGEM-T vector (Promega), the positive clones were selected and sequenced. The VLCL gene which was sequenced as correct was cleaved from pGEM-T vector by HindIII and EcoR I and cloned into pcDNA3.1/ZEO(+) vector (Invitrogen, US) to construct eukaryotic expression vector pcDNA3.1/ZEO(+) (VLCL).

3 × 10⁵ CHO-K1 cells were inoculated to 3.5 cm tissue culture dishes, and transfected when the cells were cultured to 90-95% fusion: 10µg plasmids (4µg plasmid cDNA3.1(+)(VHCH), 6µg plasmid pcDNA3.1/ZEO(+) (VLCL)) and 20µl Lipofectamine2000 Reagent (Invitrogen) were dissolved in 500µl serum-free DMEM medium, respectively, laid still at room temperature for 5 min, the above 2 liquids were mixed and incubated at room temperature for 20min to form DNA-liposome complex. During this period, the serum containing medium in the culture dishes was replaced by 3 ml serum-free DMEM medium, then the formed DNA-liposome complex was added to a plate and cultured for 4 hours (h) in CO₂ incubator, then 2ml DMEM complete medium containing 10% serum was supplemented, and cultured in CO₂ incubator continuously. 24h after transfection, the cells were transferred to selection medium containing 600µg/ml G418 and 250µg/ml Zeocin to select resistant clones. Cell culture supernatants were taken to select highly expressed clones by ELISA: the goat anti-human IgG (Fc) were coated on ELISA plate, overnight at 4°C, and blocked at 37°C for 2h using 2% BSA-PBS, the resistant clone culture supernatants to be tested or standard sample (Human myeloma IgG1, κ) were added and warm incubated at 37°C for 2h, HRP-goat anti-human IgG(κ) were added for combining reaction, warm incubated at 37°C for 1h and TMB was added and effected at 37°C for 5 min, and finally H₂SO₄ was used to stop the reaction, A₄₅₀ value was measured. The highly expressed clones obtained by selection were enlarged cultured using serum-free medium, the chimeric antibody c4C8 was isolated and purified by Protein A affinity column (GE company). The purified antibody was dialyzed using PBS, and ultraviolet absorption method was used for quantification.

### Example 3. Construction of humanized antibody 4C8

### Homology Modeling of the Three-dimensional Structure of Murine-derived 4C8 Monoclonal Antibody Variable Region (Fv)

The three dimensional structure of murine-derived 4C8 monoclonal antibody variable region was built by Insight II software package from Accelrys company. Firstly, the template proteins for 4C8 heavy chain and light chain variable regions proteins were searched in Protein Data Bank (PDB) by BLAST program, respectively. An antibody 1A4J which has the highest homology is selected as modeling template for 4C8, for modeling the three dimensional structure of 4C8 using Insight II program, as shown in Figure 1.

### Design and Construction of Humanized 4C8 Antibody

Genbank database was searched for human-derived templates that are most similar to 4C8 light chain and heavy chain variable regions using BLAST program. The human-derived antibody having the highest homology with 4C8 heavy chain variable region is human antibody AAC 18206 (GenBank No. AAC18206), with the similarity of 68%, and the human-derived antibody having the highest homology with 4C8 light chain variable region is BAC01734 (GenBank No. BAC01734), with the similarity of 80%. Accordingly, AAC 18206 and BAC01734 were used as the humanized templates for 4C8 heavy chain and light chain, respectively. Firstly, 4C8 heavy chain and light chain CDR regions were grafted into humanized templates AAC 18206 and BAC01734 respectively to form CDR grafted antibodies, h4C8Ha and h4C8La for heavy chain and light chain, respectively. The amino acid sequences for h4C8Ha and h4C8La variable regions are shown in Figure 2. Humanized antibody heavy chain and light chain variable genes (h4C8VHa and h4C8VLa) were synthesized by total gene synthesis, then h4C8VHa gene and pGEM-T/CH vector were used as template to synthesize humanized antibody heavy chain gene by overlapping PCR, the reaction conditions were: 95°C for 15 min; 94°C for 50 sec, 58°C for 50 sec, 72°C for 50 sec, for 30 cycles; 72°C for 10 min. Besides, this humanized heavy chain gene was made restriction enzyme site HindIII and signal peptide gene sequence at 5' end and translation stop code TAA and restriction enzyme site EcoRI at 3' end. The sequence of signal peptide is shown in SEQ ID NO:13
(ATGGATTTTCAGGTGCAGATTTTCAGCTTCCTGCTAATCAGTGCCTCAGTCATAATATC CAGAGGA). Finally, the PCR amplification products were separated by agarose gel electrophoresis and the band of interest was recovered and cloned into pGEMT vector to select positive clone and sequenced. The clone with correct sequence was selected and digested by Hind III and EcoRI, after agarose gel electrophoresis, the humanized antibody heavy chain fragment H4C8VHaCH was recovered and ligated with plasmid pcDNA3.1(+)(Invitrogen, USA) digested by HindIII and EcoRI, to construct humanized heavy chain eukaryotic expression vector pcDNA3.1 (+) (h4C8VHaCH).

Humanized antibody light chain gene was synthesized by overlapping PCR using h4C8VLa gene and pGEM-T/CL vector as template, the reaction conditions were: 95°C for 15 min; 94°C for 50 sec, 58°C for 50 sec, 72°C for 50 sec, for 30 cycles; 72°C for 10 min. The PCR product thus obtained is h4C8VLaCL, the 5' end of which contains restriction enzyme site HindIII and signal peptide gene sequence and 3' end of which contains translation stop code TAA and restriction enzyme site EcoRI. The sequence of signal peptide is shown in SEQ ID NO:13
(ATGGATTTTCAGGTGCAGATTTTCAGCTTCCTGCTAATCAGTGCCTCAGTCATAATATC CAGAGGA). The clone with correct sequence was selected and digested by Hind III and EcoRI, after agarose gel electrophoresis, the humanized antibody light chain fragment H4C8VLaCL was recovered and ligated with plasmid pcDNA3.1(+) vector (Invitrogen, USA) digested also by HindIII and EcoRI, to construct humanized light chain eukaryotic expression vector pcDNA3.1/ZEO(+)(h4C8VLaCL).

COS-1 cells (ATCC CRL 1650) at 0.8 × 10⁵/well were inoculated to 24-well tissue culture plate, and cultured to 90-95% fusion using 10% FCS containing RPMI1640/DMEM mixed medium (16/DM medium) to perform transfection: 1µg plasmid (0.6µg light chain expression vector; 0.4µg heavy chain expression vector) and 2µl Lipofectamine2000 Reagent were dissolved in 50µl serum-free 16/DM medium, respectively, laid still at room temperature for 5 min, the above-mentioned 2 liquids were mixed and incubated at room temperature for 20min to form DNA-liposome complex. During this period, the serum containing medium in the 24-well culture plate was replaced by 0.5 ml serum-free 16/DM medium, then the formed DNA-liposome complex was added to the well and cultured in CO₂ incubator for 4 hours (h), then 0.5 ml 16/DM medium containing 20% FCS was supplemented, and cultured in CO₂ incubator continuously. After 72h, the culture supernatants were taken for analysis, the content of antibody in the culture supernatants was determined by ELISA: the goat anti-human IgG (Fc) were coated in ELISA plate, overnight at 4°C, and blocked at 37°C for 2h using 2% BSA-PBS, the culture supernatants to be tested and standard sample (Human myeloma IgG1,κ) were added and incubated at 37°C for 2h, HRP-goat anti-humanκ were added for combining reaction, incubated at 37°C for 1h and TMB was added and effected at 37°C for 5 min, and finally H₂SO₄ was used to stop the reaction, the OD₄₅₀ value was measured.

Human KG-1a cells were re-suspended in 2%FCS-PBS to 1×10⁶ cells/ml, adding COS cells culture supernatants transfecting humanized antibody with different dilution degree, respectively, they were incubated at 4°C for 60min, then cells were washed twice with 2% FCS-PBS, and then FITC-goat anti-human IgG (H+L) was added to the cells and incubated at 4°C for 60min, after washing, cells were analyzed by Flow Cytometry (FCM) and calculated for fluorescence intensity. The result shows that to compared with c4C8 chimeric antibody, the humanized antibody composed of h4C8Ha and h4C8La (h4C8Ha/h4C8La) almost loses its activity completely (Figure. 3). Hence, to obtain humanized antibody with high affinity, we need further to perform analysis and back-mutation on the murine-derived residues in FR region which may possibly affect the 4C8 antibody binding activity.

By analyzing the three-dimensional structure of the modeled 4C8 monoclonal antibody variable region, we have discovered nine framework residues, L3Leu, L4Leu, L46Leu, H2Ile, H46Lys, H68Ala, H69Leu, H82aAsn, and H91Phe within 5A° around the CDR regions might affect the CDR conformation of the original antibody while differ from the corresponding position of humanized template. Remaining these murine-derived amino acid residues in the constructed CDR grafted antibody can yield humanized antibody (h4C8Hb/h4C8Lb). The amino acid sequences for the variable regions of h4C8Hb and h4C8Lb are shown in Figure 2. SEQ ID NO:5 and SEQ ID NO:6 show the nucleotide sequence and amino acid sequence of h4C8Hb, respectively. SEQ ID NO:7 and SEQ ID NO:8 show the nucleotide sequence and amino acid sequence of h4C8Lb, respectively. Three CDR regions amino acid sequences of h4C8Hb are (see, H4C8VHb of Figure. 2-1): HCDR1 (GYTFTNYGMN, SEQ ID NO:14), HCDR2 (WINTNTGEPKYAEEFKG, SEQ ID NO:15), HCDR3 (GYGNYARGAWLAY, SEQ ID NO:16), respectively; Three CDR regions amino acid sequences of h4C8Lb are (see, H4C8VLb of Figure. 2-2): LCDR1 (RSSQTIVHSNGNTYLE, SEQ ID NO:17), LCDR2 (QVSNRFS, SEQ ID NO:18), LCDR3 (FQGSHVPRT, SEQ ID NO:19), respectively. Humanized antibody heavy and light chain variable regions gene (h4C8VHb/h4C8VLb) were synthesized using overlapping PCR method, respectively, and the same method as to humanized antibody (h4C8Ha/h4C8La) were used to construct light chain expression vector pcDNA3.1/ZEO(+) (h4C8VLbCL) and heavy chain expression vector pcDNA3.1 (+) (h4C8VHbCH). Then light and heavy expression vectors were cotransfected into COS-1 cells. Flow cytometry assay was performed to determine the antigen-binding activities of the antibody, showing its binding ability to KG-1a is similar to 4C8 chimeric antibody, and thus this humanized antibody (h4C8Hb/h4C8Lb) is designated as h4C8.

### Example 4. Stable expression and purification of humanized antibody

3 × 10⁵ CHO-K1 cells (ATCC CRL-9618) were inoculated to 3.5cm tissue culture dishes, and transfected when the cells were cultured to 90-95% fusion: 10µg plasmids (4µg plasmid pcDNA3.1(+)(Vh4C8HbCH), 6µg plasmid pcDNA3.1/ZEO(+) (Vh4C8LbCL)) and 20µl Lipofectamine2000 Reagent (Invitrogen) were dissolved in 500µl serum- free DMEM medium, respectively, laid still at room temperature for 5 min, the above-mentioned two liquids were mixed and incubated at room temperature for 20min to form DNA-liposome complex. During this period, the serum containing medium in the culture dishes was replaced by 3 ml serum-free DMEM medium, then the formed DNA-liposome complex was added to a plate and cultured for 4 hours (h) in CO₂ incubator, then 2 ml DMEM complete medium containing 10% serum was supplemented, and cultured in CO₂ incubator continuously. 24h after transfection, the cell were transferred to selection medium containing 600µg/ml G418 and 250µg/ml Zeocin to select resistant clones. Cell culture supernatants were taken to select highly expressed clones: the goat anti-human IgG (Fc) were coated on ELISA plate, overnight at 4°C, and blocked at 37°C for 2h using 2% BSA-PBS, the culture supernatant of resistant clones to be tested or standard sample (Human myeloma IgG1, κ) were added and warm incubated at 37°C for 2h, HRP-goat anti-human IgG(κ) were added for combining reaction, warm-incubated at 37°C for 1h and TMB was added and effected at 37°C for 5 min, and finally H₂SO₄ was used to stop the reaction, A₄₅₀ value was measured. The highly expressed clones obtained by selection was enlarged cultured using serum-free medium, the humanized antibody h4C8 was isolated and purified by Protein A affinity column (GE company). The purified antibody was dialyzed using PBS, and ultraviolet absorption method was used for quantification.

### Example 5. Competitive Inhibition Experiment

A fixed subsaturating concentration of fluorescently labeled antibody FITC-4C8 and serial dilution of unlabeled purified antibodies were mixed respectively and then added into target KG-1a cells (1×10⁶ /ml), and incubated at 4°C for 60 min, the cells were washed twice with 1% FCS-PBS and examined by FCM using Cellquest software for analysis. Humanized anti-CD3 monoclonal antibody hu12F6 (preparation see to Li BH, Wang H, Dai JX, Ji JJ, Qian WZ, Zhang DP, Hou S, Guo YJ. Construction and characterization of a humanized anti-human CD3 monoclonal antibody 12F6 with effective immunoregulation functions. Immunology. 2005, 116(4): 487-98*)* was used as control. Triplicate tubes were set for each concentration of the competitive antibody. The IC₅₀ values were calculated, with the maximum fluorescence intensity indicating the average fluorescence intensity obtained in the absence of competitive antibody.

The experimental result is shown in Figure 4, antibody h4C8 can completely block the binding of fluorescently labeled antibody FITC-4C8 to KG-1a, their IC₅₀ values were about the same, suggesting that this humanized antibody possessed specificity and affinity similar to that of the original murine- derived antibody (Table 1).

**Table 1. Result of competitive inhibition analysis**

| Type of antibodies | IC₅₀ (µg/ml)^{a} | SD | n |
|---|---|---|---|
| 4C8 | 1.723 | 0.145 | 3 |
| c4C8 | 1.692 | 0.137 | 3 |
| h4C8 | 1.764 | 0.158 | 3 |

### Example 6. Preparation of immunomagnetic beads

Activation: 0.01mol/L NaH₂PO₄ Tween (0.05% Tween-20) solution, pH6.0 was taken as activated buffer solution, 2 mg carboxyl-beads (Bangs laboratories, BioMag Carboxyl (BM570), USA) was put into 2ml centrifuge tube, and 500µl activated buffer solution was added to mix homogeneously on the vortex mixer, then the centrifuge tube was laid on a Magnetic Separation Rack, after the magnetic beads were completely adsorbed, the supernatant was extracted by mini vacuum pump; 500µl activated buffer solution was added to rewash the magnetic beads twice, then 485µl activated buffer solution was added to the magnetic beads, and 2.5mg carbodiimide (EDC, original concemation of 0.5g/ml) and N-Hydroxysuccinimide (NHS, original concernation of 0.25g/ml) were added respectively and mixed homogeneously on the vortex mixer to activate the 2 mg carboxyl group on the surface of magnetic beads for 30 min at room temperature.

Conjugation: 500µl, pH7.4, 0.01mol/L phosphate Tween (PBST, 0.05% Tween-20) solution was used as conjugated buffer, 500µl activated buffer solution was added to rewash the activated magnetic beads 3 times, then 500µl conjugated buffer was added to wash the magnetic beads twice; 475µl conjugated buffer was added to re-suspend the washed magnetic beads, and 25µl 6mg/ml anti-CD34 antibody was added, allowing the activated carboxyl group on the magnetic beads to react with the amino group of the anti-CD34 antibody at room temperature for 3h, then the antibody was conjugated to the surface of the magnetic beads, yielding the immunomagnetic beads.

Block: the conjugated magnetic beads were washed by 500µl conjugated buffer twice, and 500µl conjugated buffer containing 1% bovine serum albumin (BSA) was added to block the magnetic beads for 30 min.

Storage: the conjugated magnetic beads were washed by 500µl conjugated buffer twice, and the magnetic beads were re-suspended in 500µl pH7.4, 0.01mol/L phosphate Tween (0.05% Tween-20) solution containing 0.02% NaN₃, 0.1% BSA, and then stored in refrigerator at 4°C for use.

### Example 7. Sorting for CD34+ cell line by CD34 immunomagnetic beads

Mixing CD34+ KG-1a (human leukaemia cell) and CD34- Raji (human B lymphoma cell) *in vitro,* in the ratio of 1:10, to imitate human umbilical cord blood mononuclear cell (or mobilized peripheral blood mononuclear cell).

Preparing 0.1M PBS solution: NaCl 8g, KCl 0.2g, Na₂HPO₄-12H₂O 3.488g, KH₂PO₄ 0.2g, adding Milli-Q water to 1000ml, adjusting to pH 7.4, sterilizing using 0.22µm filter, storing at 4°C.

Preparing immunomagnetic beads sorting buffer: 0.1M PBS solution containing 2 mM EDTA and 0.5% BSA.

Adding sorting buffer in the ratio of 90µl /10⁷ cells to re-suspend the above-mentioned mixed cells;

Adding CD34 immunomagnetic beads into the above-mentioned cell suspension in the ratio of 10µl /10⁷ cells, mixing and incubating at 4-8°C for 15 min;

Adding the sorting buffer in the ratio of 1000µl/10⁷ cells (1000µl is also applicable for cells less than 10⁷) to re-suspend the cells; laying the centrifuge tube in the magnetic field, and removing the supernatants;

Repeating the above-mentioned steps twice;

Re-suspending the cell precipitates using 500µl sorting buffer, yielding CD34+ KG-1a cells.

The cells were stained with CD34-FITC (8G12, BD Bioscience, 340668, having no competitive effect with sorting antibody), the purity of the cells obtained by sorting were identified as more than 95%; the sorting cells were counted, with the yeild of sorting CD34+ cells of more than 90%; the result is shown in Figure 5.

### Example 8. Sorting for human umbilical cord blood stem cells by CD34 immunomagnetic beads

CD34+ blood stem cells were sorted from human umbilical cord blood.

Preparing 0.1M PBS solution: NaCl 8g, KCl 0.2g, Na₂HPO₄-12H₂O 3.488g, KH₂PO₄ 0.2g, adding Milli-Q water to 1000ml, adjusting to pH 7.4, sterilizing using 0.22µm filter, storing at 4°C.

Preparing immunomagnetic beads sorting buffer: 0.1M PBS solution containing 2 mM EDTA and 0.5% BSA.

Isolating mononuclear cell from healthy maternal umbilical cord blood using Ficoll.

Adding sorting buffer in the ratio of 90µl /10⁷ cells to re-suspend the mononuclear cell;

Adding CD34 immunomagnetic beads into the above-mentioned cell suspension in the ratio of 10µl /10⁷ cells, mixing and incubating at 4-8°C for 15 min;

Adding the sorting buffer in the ratio of 1000µl/10⁷ cells (1000µl is also applicable for cells less then 10⁷ ) to re-suspend cells; laying the centrifuge tube in the magnetic field, and removing the supernatants;

Repeating the above-mentioned steps twice;

Re-suspending the cell precipitates using 500µl sorting buffer, yielding CD34+ blood stem cells.

The cells were stained by CD34-FITC (8G12, BD Bioscience, 340668, having no competitive effect with sorting antibody), the purity of the cells obtained by sorting were identified as more than 95%; the sorting cells were counted, with the yeild of sorting CD34+ cells of more than 90%; the result is shown in Figure 6.

The results indicate the purity and yield obtained by sorting the umbilical cord blood stem cells using the CD34 immunomagnetic beads we prepared has meet the requirement of clinical application, and is expected to be used in clinical transplantation of marrow blood stem cells.

### Example 9. Competitive inhibition test for humanized anti-CD34 antibody and murine-derived antibody My10.

A subsaturating concentration of FITC-CD34 (My10) (BD, 348053) and serial dilution of unlabeled purified antibodies h4C8 and unlabeled My10 (mouse anti-human CD34 hybridoma cells (ATCC, HB8483, anti-My10 clone), were cultured by serum-free medium Hybridoma-SFM (GIBCO, 12045). After purification by rProteinA affinity chromatography (GE), the culture supernatants were mixed respectively and then added into target cells KG-1a (1×10⁶/ml), and incubated at 4°C for 60 min, the cells were washed twice with 1% FCS-PBS and examined by FCM using Cellquest software for analysis. Humanized anti-CD3 monoclonal antibody hu12F6 (preparation see to Li BH, Wang H, Dai JX, Ji JJ, Qian WZ, Zhang DP, Hou S, Guo YJ. Construction and characterization of a humanized anti-human CD3 monoclonal antibody 12F6 with effective immunoregulation functions. Immunology. 2005, 116(4): 487-98*)* was used as control. Triplicate tubes were set for each concentration of the competitive antibody. The IC₅₀ values were calculated, with the maximum fluorescence intensity indicating the average fluorescence intensity obtained in the absence of competitive antibody. Repeating this test for 3 times, and gaining statistical the result, see to Table 2.

The test result is shown in Figure 7, the IC₅₀ of the binding of fluorescently labeled antibody FITC-My10 to KG-1a cells blocked by antibody h4C8 was smaller than by murine-derived monoclonal antibody My10 (p < 0.05, t-test), hence the affinity of h4C8 is better than murine-derived monoclonal antibody My10.

**Table 2. Result of competitive inhibition analvsis**

| Type of antibodies | IC₅₀(µg/ml) | *X̅* ± *SD* | p |
|---|---|---|---|
| My10 | 8.67 | 8.69±0.53 | 1.54×10-5 |
| | 9.23 | | |
| | 8.18 | | |
| h4C8 | 1.11 | 1.09±0.06 | |
| | 1.14 | | |
| | 1.02 | | |

### Example 10. Comparative test for sorting umbilical cord blood stem cells after the conjugation to magnetic beads by humanized anti-CD34 antibody and murine-derived antibody My10

The mouse anti-human CD34 hybridoma cells (ATCC, HB8483, anti-My10 clone) were cultured using serum-free medium Hybridoma-SFM (GIBCO, 12045). The culture supernatants were purified by rProteinA affinity chromatography (GE), yielding anti-CD34 monoclonal antibody (My10) that meets the requirement of magnetic beads preparing conditions.

My10 and h4C8 were conjugated with magnetic beads according to the method of Example 6, to prepare CD34 nano immunomagnetic beads.

Human umbilical cord blood stem cells were sorted by My10 and h4C8 nano immunomagnetic beads according to the method of Example 8. The positive cells obtained by sorting were counted by blood counting plate, and the yield of the cells obtained by sorting by the two magnetic beads were calculated; the positive cells obtained by sorting were stained by FITC-anti-CD34 (BD, 348053, 8G12), and the purity of the cells obtained by sorting by the two magnetic beads were identified, with the stained results shown in Figure 8 (peak at left side is the test result before sorting, and peak at right side is the test result after sorting).

The result of comparative test was shown in Table 3.

**Table 3.**

| | My10 magnetic beads | h4C8 magnetic beads |
|---|---|---|
| Yield (%) | 78% | 91% |
| Purity (%) | 91% | 95% |

### INDUSTRIAL UTILITY

The present humanized anti-CD34 antibody retains the affinity and specificity of original murine-derived antibody. The antibody can be conjugated with nano magnetic materials to prepare immunomagnetic beads to sort marrow hemopoietic stem cells. It can effectively reduce the incidence rate of HAMA and improve the security of clinical transplantation of hemopoietic stem cells and can be used in treatment of some malignant hematologic diseases and solid tumors. The present humanized anti-CD34 antibody is obtained by genetic engineering technology.

## Claims

1. A humanized anti-human CD34 antibody, said antibody comprises at least one antigen binding site, said antigen binding site at least comprising:
• a first variable region having an amino acid sequence as shown in amino acids 1-122 of SEQ ID NO: 6; and
• a second variable region having an amino acid sequence as shown in amino acids 1-113 of SEQ ID NO: 8.

2. The humanized anti-human CD34 antibody of claim 1, wherein said antibody comprises:
1) an immunoglobulin heavy chain or fragment thereof, comprising said first variable region and human immunoglobulin heavy chain constant region or fragment thereof; and
2) an immunoglobulin light chain or fragment thereof, comprising said second variable region and human immunoglobulin light chain constant region or fragment thereof.

3. The humanized anti-human CD34 antibody of any of claims 1-2, wherein said human immunoglobulin heavy chain constant region or fragment thereof is type γ; said human immunoglobulin light chain constant region or fragment thereof is type κ or type λ.

4. A nucleotide molecule encoding the humanized anti-human CD34 antibody of any of claims 1-3, said nucleotide molecule at least comprising a nucleotide sequence encoding the first variable region according to claim 1 as shown in nucleotides 1-366 of SEQ ID NO: 5; and a nucleotide sequence encoding the second variable region according to claim 1 as shown in nucleotides 1- 339 of SEQ ID NO: 7.

5. The nucleotide molecule of claim 4, comprising the following sequences:
a) a nucleotide sequence encoding immunoglobulin heavy chain or fragment thereof, comprising a nucleotide sequence encoding said first variable region and a nucleotide sequence encoding human immunoglobulin heavy chain constant region or fragment thereof; and
b) a nucleotide sequence encoding immunoglobulin light chain or fragment thereof, comprising a nucleotide sequence encoding said second variable region and a nucleotide sequence encoding human immunoglobulin light chain constant region or fragment thereof.

6. The nucleotide molecule of claim 5, wherein said nucleotide sequence of heavy chain constant region is shown in SEQ ID NO: 1; said nucleotide sequence of light chain constant region is shown in SEQ ID NO: 3.

7. A bead comprising the humanized anti-human CD34 antibody of any of claims 1-3.

8. The bead of claim 7, wherein said bead is an immunomagnetic bead.

9. A use of the humanized anti-human CD34 antibody of any of claims 1-3 in sorting marrow hemopoietic stem/progenitor cells.

10. A preparation method of the humanized anti-human CD34 antibody of any of claims 1-3, comprising the following steps:
1) transforming an expression vector using the nucleotide molecule of claims 4-6, and transforming a host cell using the obtained expression vector;
2) culturing said host cell;
3) collecting, separating and purifying the humanized anti-human CD34 antibody expressed by said host cell.

## Patentansprüche

1. Ein humanisierter anti-human CD34 Antikörper, wobei der Antikörper wenigstens eine Antigenbindungsstelle umfasst, wobei die Antigenbindungsstelle wenigstens umfasst:
• eine erste variable Region mit einer Aminosäuresequenz wie in den Aminosäuren 1 - 122 der SEQ ID No: 6; und
• eine zweite variable Region mit einer Aminosäuresequenz wie in den Aminosäuren 1 - 113 der SEQ ID No: 8.

2. Der humanisierte anti-human CD34 Antikörper nach Anspruch 1, wobei der Antikörper umfasst:
1) eine Immunglobulin schwere Kette oder ein Fragment davon, umfassend die erste variable Region und die humane Immunglobulin schwere Kette konstante Regionen oder ein Fragment davon; und
2) eine Immunglobulin leichte Kette oder ein Fragment davon, umfassend die zweite variable Region und eine humane Immunglobulin leichte Kette konstante Region oder ein Fragment davon.

3. Der humanisierte Anti-Human CD34 Antikörper nach einem der Ansprüche 1 - 2, wobei die humane Immunglobulin schwere Kette konstante Region oder ein Fragment davon vom Typ γ ist, wobei die humane Immunglobulin leichte Kette kontante Region oder ein Fragment davon vom Typ κ oder Typ λ ist.

4. Ein Nukleotidmolekül kodierend für den humanisierten anti-human CD34 Antikörper nach einem der Ansprüche 1 - 3, wobei das Nukleotidmolekül wenigstens eine Nukleotidsequenz kodierend für die erste variable Region nach Anspruch 1 wie in den Nukleotiden 1 - 366 von SEQ ID No: 5 angegeben kodiert; und eine Nuleotidsequenz kodierend für die zweite variable Region nach Anspruch 1 wie in den Nukleotiden 1 - 339 von Sequenz SEQ ID No: 7 angegeben kodiert.

5. Das Nukleotidmolekül nach Anspruch 4 umfassend die folgenden Sequenzen:
a) eine Nukleotidsequenz kodierend für Immunglobulin schwere Kette oder ein Fragment davon, umfassend eine Nukleotidsequenz kodierend für die erste variable Region und eine Nukleotidsequenz kodierend für die humane Immunglobulin schwere Kette konstante Region oder ein Fragment davon; und
b) eine Nukleotidsequenz kodierend für die Immunglobulin leichte Kette oder ein Fragment davon, umfassend eine Nukleotidsequenz kodierend für die zweite variable Region und eine Nukleotidsequenz kodierend für humane Immunglobulin leichte Kette konstante Region oder ein Fragment davon.

6. Das Nukleotidmolekül nach Anspruch 5, wobei die Nukleotidsequenz der schweren Kette konstanten Region in SEQ ID No: 1 angegeben ist; wobei die Nukleotidsequenz der leichten Kette kontanten Region in SEQ ID No: 3 angegeben ist.

7. Ein Kügelchen umfassend einen humanisierten anti-human CD34 Antikörper nach einem der Ansprüche 1 - 3.

8. Das Kügelchen nach Anspruch 7, wobei das Kügelchen ein immunomagnetisches Kügelchen ist.

9. Eine Verwendung von einem humanisierten anti-human CD34 Antikörper nach einem der Ansprüche 1 - 3 in der Trennung markblutbildender Stamm/Vorläuferzellen

10. Ein Herstellungsverfahren für einen humanisierten anti-human CD34 Antikörper nach einem der Ansprüche 1 - 3 umfassend die folgenden Schritte:
1) Transformation eines Expressionsvektors unter Verwendung des Nukleotidmoleküls nach den Ansprüchen 4 - 6, und Transformation einer Wirtszelle unter Verwendung des erhaltenen Expressionsvektors;
2) Kultivierung der Wirtszelle;
3) Erhalt, Trennung und Reinigung des humanisierten anti-human CD34 Antikörpers, der in der Wirtszelle exprimiert wurde.

## Revendications

1. Anticorps anti-CD34 humain humanisé, ledit anticorps comprenant au moins un site de liaison d'un antigène, ledit site de liaison d'un antigène comprenant au moins :
une première région variable ayant une séquence d'acides aminés telle que représentée par les acides aminés 1 à 122 de SEQ ID NO : 6 ; et
une seconde région variable ayant une séquence d'acides aminés telle que représentée par les acides aminés 1 à 113 de SEQ ID NO : 8.

2. Anticorps anti-CD34 humain humanisé selon la revendication 1, où ledit anticorps comprend :
1) une chaîne lourde d'immunoglobuline ou un fragment de celle-ci, comprenant ladite première région variable et une région constante de chaîne lourde d'immunoglobuline humaine ou un fragment de celle-ci ; et
2) une chaîne légère d'immunoglobuline ou un fragment de celle-ci, comprenant ladite seconde région variable et une région constante de chaîne légère d'immunoglobuline humaine ou un fragment de celle-ci.

3. Anticorps anti-CD34 humain humanisé selon l'une quelconque des revendications 1 ou 2, où ladite région constante de chaîne lourde d'immunoglobuline humaine ou un fragment de celle-ci est de type γ ; ladite région constante de chaîne légère d'immunoglobuline humaine ou un fragment de celle-ci est de type κ ou de type λ.

4. Molécule nucléotidique codant pour l'anticorps anti-CD34 humain humanisé selon l'une quelconque des revendications 1 à 3, ladite molécule nucléotidique comprenant au moins une séquence nucléotidique codant pour la première région variable selon la revendication 1 telle que représentée par les nucléotides 1 à 366 de SEQ ID NO : 5 ; et une séquence nucléotidique codant pour la seconde région variable selon la revendication 1 telle que représentée par les nucléotides 1 à 339 de SEQ ID NO : 7.

5. Molécule nucléotidique selon la revendication 4, comprenant les séquences suivantes :
a) une séquence nucléotidique codant pour une chaîne lourde d'immunoglobuline ou un fragment de celle-ci, comprenant une séquence nucléotidique codant pour ladite première région variable et une séquence nucléotidique codant pour une région constante de chaîne lourde d'immunoglobuline humaine ou un fragment de celle-ci ; et
b) une séquence nucléotidique codant pour une chaîne légère d'immunoglobuline ou un fragment de celle-ci, comprenant une séquence nucléotidique codant pour ladite seconde région variable et une séquence nucléotidique codant pour une région constante de chaîne légère d'immunoglobuline humaine ou un fragment de celle-ci.

6. Molécule nucléotidique selon la revendication 5, où ladite séquence nucléotidique de la région constante de chaîne lourde est représentée par SEQ ID NO : 1 ; ladite séquence nucléotidique de la région constante de chaîne légère est représentée par SEQ ID NO : 3.

7. Bille comprenant l'anticorps anti-CD34 humain humanisé selon l'une quelconque des revendications 1 à 3.

8. Bille selon la revendication 7, où ladite bille est une bille immunomagnétique.

9. Utilisation de l'anticorps anti-CD34 humain humanisé selon l'une quelconque des revendications 1 à 3 dans le tri de cellules souches/progénitrices hématopoïétiques de la moelle.

10. Procédé de préparation de l'anticorps anti-CD34 humain humanisé selon l'une quelconque des revendications 1 à 3, comprenant les étapes suivantes :
1) la transformation d'un vecteur d'expression en utilisant la molécule nucléotidique selon les revendications 4 à 6, et la transformation d'une cellule hôte en utilisant le vecteur d'expression obtenu ;
2) la culture de ladite cellule hôte ;
3) le recueil, la séparation et la purification de l'anticorps anti-CD34 humain humanisé exprimé par ladite cellule hôte.


### SEQUENCE LISTING

<110> Shanghai Guojian Bio-Tech Institute
<120> Humanized anti-human CD34 antibody, the preparation method and uses thereof
<130> P2404EP00
<140> 08865354.8
   <141> 2010-06-30
<150> CN200710094456.9
   <151> 2007-12-13
<150> CN200810043016.5
   <151> 2008-01-08
<160> 29
<170> PatentIn version 3.5
<210> 1
   <211> 990
   <212> DNA
   <213> Artificial Sequence
<220>
   <223> Nucleotide sequence coding the human immunoglobulin heavy chain constant region
<400> 1
   gctagcacca agggcccatc ggtcttcccc ctggcaccct cctccaagag cacctctggg      60
   ggcacagcgg ccctgggctg cctggtcaag gactacttcc ccgaaccggt gacggtgtcg     120
   tggaactcag gcgccctgac cagcggcgtg cacaccttcc cggctgtcct acagtcctca     180
   ggactctact ccctcagcag cgtggtgacc gtgccctcca gcagcttggg cacccagacc     240
   tacatctgca acgtgaatca caagcccagc aacaccaagg tggacaagaa agttgagccc     300
   aaatcttgtg acaaaactca cacatgccca ccgtgcccag cacctgaact cctgggggga     360
   ccgtcagtct tcctcttccc cccaaaaccc aaggacaccc tcatgatctc ccggacccct     420
   gaggtcacat gcgtggtggt ggacgtgagc cacgaagacc ctgaggtcaa gttcaactgg     480
   tacgtggacg gcgtggaggt gcataatgcc aagacaaagc cgcgggaaga gcagtacaac     540
   agcacgtacc gtgtggtcag cgtcctcacc gtcctgcacc aggactggct gaatggcaag     600
   gagtacaagt gcaaggtctc caacaaagcc ctcccagccc ccatcgagaa aaccatctcc     660
   aaagccaaag ggcagccccg agaaccacag gtgtacaccc tgcccccatc ccgggatgag     720
   ctgaccaaga accaggtcag cctgacctgc ctggtcaaag gcttctatcc cagcgacatc     780
   gccgtggagt gggagagcaa tgggcagccg gagaacaact acaagaccac gcctcccgtg     840
   ctggactccg acggctcctt cttcctctac agcaagctca ccgtggacaa gagcaggtgg     900
   cagcagggga acgtcttctc atgctccgtg atgcatgagg ctctgcacaa ccactacacg     960
   cagaagagcc tctccctgtc tcccggtaaa                                      990
<210> 2
   <211> 330
   <212> PRT
   <213> Artificial Sequence
<220>
   <223> The human immunoglobulin heavy chain constant region
<400> 2
<210> 3
   <211> 318
   <212> DNA
   <213> Artificial Sequence
<220>
   <223> Nucleotide sequence coding the human immunoglobulin light chain constant region
<400> 3
   actgtggctg caccatctgt cttcatcttc ccgccatctg atgagcagtt gaaatctgga 60
   actgcctctg ttgtgtgcct gctgaataac ttctatccca gagaggccaa agtacagtgg 120
   aaggtggata acgccctcca atcgggtaac tcccaggaga gtgtcacaga gcaggacagc 180
   aaggacagca cctacagcct cagcagcacc ctgacgctga gcaaagcaga ctacgagaaa 240
   cacaaagtct acgcctgcga agtcacccat cagggcctga gctcgcccgt cacaaagagc 300
   ttcaacaggg gagagtgt 318
<210> 4
   <211> 106
   <212> PRT
   <213> Artificial Sequence
<220>
   <223> The human immunoglobulin light chain constant region
<400> 4
   Thr Val Ala Ala Pro Ser Val Phe Ile Phe Pro Pro Ser Asp Glu Gln
   1               5                   10                  15
   Leu Lys Ser Gly Thr Ala Ser Val Val Cys Leu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Val Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu Cys
               100                 105
<210> 5
   <211> 1356
   <212> DNA
   <213> Artificial Sequence
<220>
   <223> Nucleotide sequence coding humanized h4C8 antibody heavy chain
<400> 5
   cagatccaac tcgtccagtc cggctcagag ctaaagaaac ccggagccag tgtaaaggtg 60
   agctgcaaag catctgggta cacctttaca aactatggta tgaattgggt tcgccaagcg 120
   ccaggccagg gactgaagtg gatggggtgg ataaacacga atactggtga accgaaatac 180
   gctgaggagt tcaagggccg attcgcctta tccttggaca cctcagtctc gacagcatat 240
   cttcaaatta acagtctcaa agcggaggat acggctgtat acttttgtgc ccggggatat 300
   gggaattacg caagaggtgc gtggctcgct tattggggcc agggaactct ggtgaccgtt 360
   tcttctgcta gcaccaaggg cccatcggtc ttccccctgg caccctcctc caagagcacc 420
   tctgggggca cagcggccct gggctgcctg gtcaaggact acttccccga accggtgacg 480
   gtgtcgtgga actcaggcgc cctgaccagc ggcgtgcaca ccttcccggc tgtcctacag 540
   tcctcaggac tctactccct cagcagcgtg gtgaccgtgc cctccagcag cttgggcacc 600
   cagacctaca tctgcaacgt gaatcacaag cccagcaaca ccaaggtgga caagaaagtt 660
   gagcccaaat cttgtgacaa aactcacaca tgcccaccgt gcccagcacc tgaactcctg 720
   gggggaccgt cagtcttcct cttcccccca aaacccaagg acaccctcat gatctcccgg 780
   acccctgagg tcacatgcgt ggtggtggac gtgagccacg aagaccctga ggtcaagttc 840
   aactggtacg tggacggcgt ggaggtgcat aatgccaaga caaagccgcg ggaagagcag 900
   tacaacagca cgtaccgtgt ggtcagcgtc ctcaccgtcc tgcaccagga ctggctgaat 960
   ggcaaggagt acaagtgcaa ggtctccaac aaagccctcc cagcccccat cgagaaaacc 1020
   atctccaaag ccaaagggca gccccgagaa ccacaggtgt acaccctgcc cccatcccgg 1080
   gatgagctga ccaagaacca ggtcagcctg acctgcctgg tcaaaggctt ctatcccagc 1140
   gacatcgccg tggagtggga gagcaatggg cagccggaga acaactacaa gaccacgcct 1200
   cccgtgctgg actccgacgg ctccttcttc ctctacagca agctcaccgt ggacaagagc 1260
   aggtggcagc aggggaacgt cttctcatgc tccgtgatgc atgaggctct gcacaaccac 1320
   tacacgcaga agagcctctc cctgtctccc ggtaaa 1356
<210> 6
   <211> 452
   <212> PRT
   <213> Artificial Sequence
<220>
   <223> humanized h4C8 antibody heavy chain
<400> 6
   Gln Ile Gln Leu Val Gln Ser Gly Ser Glu Leu Lys Lys Pro Gly Ala
   1 5 10 15
   Ser Val Lys Val Ser Cys Lys Ala Ser Gly Tyr Thr Phe Thr Asn Tyr
   20 25 30
   Gly Met Asn Trp Val Arg Gln Ala Pro Gly Gln Gly Leu Lys Trp Met
   35 40 45
   Gly Trp Ile Asn Thr Asn Thr Gly Glu Pro Lys Tyr Ala Glu Glu Phe
   50 55 60
   Lys Gly Arg Phe Ala Leu Ser Leu Asp Thr Ser Val Ser Thr Ala Tyr
   65 70 75 80
   Leu Gln Ile Asn Ser Leu Lys Ala Glu Asp Thr Ala Val Tyr Phe Cys
                   85                  90                  95
   Ala Arg Gly Tyr Gly Asn Tyr Ala Arg Gly Ala Trp Leu Ala Tyr Trp
               100                 105                 110
   Gly Gln Gly Thr Leu Val Thr Val Ser Ser Ala Ser Thr Lys Gly Pro
           115                 120                 125
   Ser Val Phe Pro Leu Ala Pro Ser Ser Lys Ser Thr Ser Gly Gly Thr
       130                 135                 140
   Ala Ala Leu Gly Cys Leu Val Lys Asp Tyr Phe Pro Glu Pro Val Thr
   145                 150                 155                 160
   Val Ser Trp Asn Ser Gly Ala Leu Thr Ser Gly Val His Thr Phe Pro
                   165                 170                 175
   Ala Val Leu Gln Ser Ser Gly Leu Tyr Ser Leu Ser Ser Val Val Thr
               180                 185                 190
   Val Pro Ser Ser Ser Leu Gly Thr Gln Thr Tyr Ile Cys Asn Val Asn
           195                 200                 205
   His Lys Pro Ser Asn Thr Lys Val Asp Lys Lys Val Glu Pro Lys Ser
       210                 215                 220
   Cys Asp Lys Thr His Thr Cys Pro Pro Cys Pro Ala Pro Glu Leu Leu
   225                 230                 235                 240
   Gly Gly Pro Ser Val Phe Leu Phe Pro Pro Lys Pro Lys Asp Thr Leu
                   245                 250                 255
   Met Ile Ser Arg Thr Pro Glu Val Thr Cys Val Val Val Asp Val Ser
   260 265 270
   His Glu Asp Pro Glu Val Lys Phe Asn Trp Tyr Val Asp Gly Val Glu
   275 280 285
   Val His Asn Ala Lys Thr Lys Pro Arg Glu Glu Gln Tyr Asn Ser Thr
   290 295 300
   Tyr Arg Val Val Ser Val Leu Thr Val Leu His Gln Asp Trp Leu Asn
   305 310 315 320
   Gly Lys Glu Tyr Lys Cys Lys Val Ser Asn Lys Ala Leu Pro Ala Pro
   325 330 335
   Ile Glu Lys Thr Ile Ser Lys Ala Lys Gly Gln Pro Arg Glu Pro Gln
   340 345 350
   Val Tyr Thr Leu Pro Pro Ser Arg Asp Glu Leu Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Val Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 7
   <211> 657
   <212> DNA
   <213> Artificial Sequence
<220>
   <223> Nucleotide sequence coding humanized h4C8 antibody light chain
<400> 7
   gacgtcttgc ttacccagtc ccccctctca ctaccagtaa cactgggcca accggcctcg    60
   atcagttgcc gcagctctca gacgatagtg cactccaacg gaaatactta cttagagtgg   120
   ttccaacagc gacctgggca atcaccgcgg ttgcttattt atcaggtttc gaacagattt   180
   agtggtgtcc ctgataggtt cagcggctct ggctccggga ccgactttac actcaagatc   240
   tcacgtgtag aagcagagga tgtgggtgtt tactattgtt tccaaggctc gcatgtcccc   300
   cgcacgtttg gagggggtac taaagtagaa ataaagcgaa ctgtggctgc accatctgtc   360
   ttcatcttcc cgccatctga tgagcagttg aaatctggaa ctgcctctgt tgtgtgcctg   420
   ctgaataact tctatcccag agaggccaaa gtacagtgga aggtggataa cgccctccaa   480
   tcgggtaact cccaggagag tgtcacagag caggacagca aggacagcac ctacagcctc   540
   agcagcaccc tgacgctgag caaagcagac tacgagaaac acaaagtcta cgcctgcgaa   600
   gtcacccatc agggcctgag ctcgcccgtc acaaagagct tcaacagggg agagtgt      657
<210> 8
   <211> 219
   <212> PRT
   <213> Artificial Sequence
<220>
   <223> Humanized h4C8 antibody light chain
<400> 8
   Asp Val Leu Leu Thr Gln Ser Pro Leu Ser Leu Pro Val Thr Leu Gly
   1 5 10 15
   Gln Pro Ala Ser Ile Ser Cys Arg Ser Ser Gln Thr Ile Val His Ser
   20 25 30
   Asn Gly Asn Thr Tyr Leu Glu Trp Phe Gln Gln Arg Pro Gly Gln Ser
   35 40 45
   Pro Arg Leu Leu Ile Tyr Gln Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Phe Gln Gly
   85 90 95
   Ser His Val Pro Arg Thr Phe Gly Gly Gly Thr Lys Val Glu Ile Lys
   100 105 110
   Arg Thr Val Ala Ala Pro Ser Val Phe Ile Phe Pro Pro Ser Asp Glu
   115 120 125
   Gln Leu Lys Ser Gly Thr Ala Ser Val Val Cys Leu Leu Asn Asn Phe
   130 135 140
   Tyr Pro Arg Glu Ala Lys Val Gln Trp Lys Val Asp Asn Ala Leu Gln
   145 150 155 160
   Ser Gly Asn Ser Gln Glu Ser Val Thr Glu Gln Asp Ser Lys Asp Ser
   165 170 175
   Thr Tyr Ser Leu Ser Ser Thr Leu Thr Leu Ser Lys Ala Asp Tyr Glu
   180 185 190
   Lys His Lys Val Tyr Ala Cys Glu Val Thr His Gln Gly Leu Ser Ser
   195 200 205
   Pro Val Thr Lys Ser Phe Asn Arg Gly Glu Cys
   210 215
<210> 9
   <211> 366
   <212> DNA
   <213> Artificial Sequence
<220>
   <223> Nucleotide sequence coding 4C8 heavy chain variable region
<400> 9
   cagatccagt tggtgcagtc tggacctgag ctgaagaagc ctggagagac agtcaatatc 60
   tcctgcaagg cgtctggata taccttcaca aactatggaa tgaactgggt gaagcaggct 120
   ccaggaaagg gtttaaagtg gatgggctgg ataaacacca acactggaga gccaaaatat 180
   gctgaagagt tcaagggacg gtttgccttg tctttggaaa cctctgccag cactgcctat 240
   ttgcagatca acaacctcaa aaatgaggac acggctacat atttctgtgc aaggggttat 300
   ggtaattacg cacgtggagc ctggttggct tactggggcc aagggactct ggtcactgtc 360
   tctgca 366
<210> 10
   <211> 122
   <212> PRT
   <213> Artificial Sequence
<220>
   <223> 4C8 heavy chain variable region
<400> 10
   Gln Ile Gln Leu Val Gln Ser Gly Pro Glu Leu Lys Lys Pro Gly Glu
   1               5                   10                  15
   Thr Val Asn Ile Ser Cys Lys Ala Ser Gly Tyr Thr Phe Thr Asn Tyr
               20                  25                  30
   Gly Met Asn Trp Val Lys Gln Ala Pro Gly Lys Gly Leu Lys Trp Met
           35                  40                  45
   Gly Trp Ile Asn Thr Asn Thr Gly Glu Pro Lys Tyr Ala Glu Glu Phe
       50                  55                  60
   Lys Gly Arg Phe Ala Leu Ser Leu Glu Thr Ser Ala Ser Thr Ala Tyr
   65                  70                  75                  80
   Leu Gln Ile Asn Asn Leu Lys Asn Glu Asp Thr Ala Thr Tyr Phe Cys
                   85                  90                  95
   Ala Arg Gly Tyr Gly Asn Tyr Ala Arg Gly Ala Trp Leu Ala Tyr Trp
               100                 105                 110
   Gly Gln Gly Thr Leu Val Thr Val Ser Ala
           115                 120
<210> 11
   <211> 339
   <212> DNA
   <213> Artificial Sequence
<220>
   <223> Nucleotide sequence coding 4C8 light chain variable region
<400> 11
   gatgttttgt tgacccaaac tccactctcc ctgcctgtca gtcttggaga tcaagcctcc     60
   atctcttgca gatctagtca gaccattgta catagtaatg gaaacaccta tttagaatgg    120
   ttcctgcaga aaccaggaca gtctccaaag ctcctgatct accaagtttc caaccgattt    180
   tctggggtcc cagacaggtt cagtggcagt ggatcaggga cagatttcac actcaagatc    240
   agcagagtgg aggctgagga tctgggagtt tattactgct ttcaaggttc acatgttcct    300
   cggacgttcg gtggaggcac caagctggaa atcaaacgg                           339
<210> 12
   <211> 113
   <212> PRT
   <213> Artificial Sequence
<220>
   <223> 4C8 light chain variable region
<400> 12
   Asp Val Leu Leu Thr Gln Thr Pro Leu Ser Leu Pro Val Ser Leu Gly
   1               5                   10                  15
   Asp Gln Ala Ser Ile Ser Cys Arg Ser Ser Gln Thr Ile Val His Ser
               20                  25                  30
   Asn Gly Asn Thr Tyr Leu Glu Trp Phe Leu Gln Lys Pro Gly Gln Ser
           35                  40                  45
   Pro Lys Leu Leu Ile Tyr Gln Val Ser Asn Arg Phe Ser Gly Val Pro
       50                  55                  60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Val Pro Arg Thr Phe Gly Gly Gly Thr Lys Leu Glu Ile Lys
   100 105 110
   Arg
<210> 13
   <211> 66
   <212> DNA
   <213> Artificial Sequence
<220>
   <223> Nucleotide sequence coding a signal peptide
<400> 13
   atggattttc aggtgcagat tttcagcttc ctgctaatca gtgcctcagt cataatatcc 60
   agagga 66
<210> 14
   <211> 10
   <212> PRT
   <213> Artificial Sequence
<220>
   <223> Amino acid sequence of CDR1 in the first variable reagion of humanized h4C8 antibody heavy chain
<400> 14
   Gly Tyr Thr Phe Thr Asn Tyr Gly Met Asn
   1 5 10
<210> 15
   <211> 17
   <212> PRT
   <213> Artificial Sequence
<220>
   <223> Amino acid sequence of CDR2 in the first variable reagion of humanized h4C8 antibody heavy chain
<400> 15
   Trp Ile Asn Thr Asn Thr Gly Glu Pro Lys Tyr Ala Glu Glu Phe Lys
   1 5 10 15
   Gly
<210> 16
   <211> 13
   <212> PRT
   <213> Artificial Sequence
<220>
   <223> Amino acid sequence of CDR3 in the first variable reagion of humanized h4C8 antibody heavy chain
<400> 16
   Gly Tyr Gly Asn Tyr Ala Arg Gly Ala Trp Leu Ala Tyr
   1 5 10
<210> 17
   <211> 16
   <212> PRT
   <213> Artificial Sequence
<220>
   <223> Amino acid sequence of CDR1 in the second variable reagion of humanized h4C8 antibody light chain
<400> 17
   Arg Ser Ser Gln Thr Ile Val His Ser Asn Gly Asn Thr Tyr Leu Glu
   1 5 10 15
<210> 18
   <211> 7
   <212> PRT
   <213> Artificial Sequence
<220>
   <223> Amino acid sequence of CDR2 in the second variable reagion of humanized h4C8 antibody light chain
<400> 18
   Gln Val Ser Asn Arg Phe Ser
   1 5
<210> 19
   <211> 9
   <212> PRT
   <213> Artificial Sequence
<220>
   <223> Amino acid sequence of CDR3 in the second variable reagion of humanized h4C8 antibody light chain
<400> 19
   Phe Gln Gly Ser His Val Pro Arg Thr
   1 5
<210> 20
   <211> 21
   <212> DNA
   <213> Artificial Sequence
<220>
   <223> Synthetic primer
<400> 20 21
   gtagaggtca gactgcagga c 21
<210> 21
   <211> 21
   <212> DNA
   <213> Artificial Sequence
<220>
   <223> Synthetic primer
<400> 21 21
   ctcagggaaa tagcccttga c 21
<210> 22
   <211> 24
   <212> DNA
   <213> Artificial Sequence
<220>
   <223> Synthetic primer
<400> 22 24
   agatccaggg gccagtggat agac 24
<210> 23
   <211> 23
   <212> DNA
   <213> Artificial Sequence
<220>
   <223> Synthetic primer
<400> 23 23
   ttgctgtcct gatcagtcca act 23
<210> 24
   <211> 23
   <212> DNA
   <213> Artificial Sequence
<220>
   <223> Synthetic primer
<400> 24
   tgtcgttcac tgccatcaat ctt 23
<210> 25
   <211> 23
   <212> DNA
   <213> Artificial Sequence
<220>
   <223> Synthetic primer
<400> 25
   ttgttcaaga agcacacgac tga 23
<210> 26
   <211> 36
   <212> DNA
   <213> Artificial Sequence
<220>
   <223> Synthetic primer
<400> 26
   aagcttgccg ccaccatgga ttggctgtgg aacttg 36
<210> 27
   <211> 24
   <212> DNA
   <213> Artificial Sequence
<220>
   <223> Synthetic primer
<400> 27
   gctagctgca gagacagtga ccag 24
<210> 28
   <211> 36
   <212> DNA
   <213> Artificial Sequence
<220>
   <223> Synthetic primer
<400> 28
   aagcttgccg ccaccatgaa gttgcctgtt aggctg 36
<210> 29
   <211> 40
   <212> DNA
   <213> Artificial Sequence
<220>
   <223> Synthetic primer
<400> 29
   gacagatggt gcagccacag tccgtttgat ttccagcttg 40